(19) Europäisches Patentamt
European Patent Office
Office européen des brevets

(11) **EP 3 567 601 A1**

(12) **EUROPEAN PATENT APPLICATION**

(43) Date of publication:
**13.11.2019 Bulletin 2019/46**

(51) Int Cl.:
***G16H 50/20*** *(2018.01)*

(21) Application number: **18171471.8**

(22) Date of filing: **09.05.2018**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
• **VUPPALA, Sunil Kamar
5656 AE Eindhoven (NL)**
• **PATIL, Meru Adagouda
5656 AE Eindhoven (NL)**
• **BUSSA, Nagaraju
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **PERSONALIZED RECOMMENDATIONS FOR HEALTH MANAGEMENT**

(57) The present disclosure discloses method and personalized recommendation system for providing personalized recommendation for health management. The personalized recommendation system receives information on medical history, one or more available lifestyle parameters of plurality of lifestyle parameters and one or more available vital parameters of plurality of vital parameters, associated with subject and estimates at least one of, one or more non-available lifestyle parameters and one or more non-available vital parameters based on at least one of, one or more available vital parameters and one or more available lifestyle parameters, using trained Neural Network (NN) Classifier. The personalized recommendation system predicts medicine dosage level, changes to be made in lifestyle parameters, indication to visit clinician and details on interaction between medications based on received information and estimation using trained NN classifier and provides personalized recommendation to at least one of subject and clinician associated with subject based on prediction.

FIG. 1

**Description**

FIELD OF THE INVENTION

This invention relates to the field of healthcare management and in particular to providing personalized recommendations for health management.

BACKGROUND OF THE INVENTION

Consumption of medicine has become part of our modern-day lifestyle. In some cases, over the counter medicines are consumed for smaller anomalies like cold or cough. However, in the majority of cases prescribed medicines are taken to treat an illness. Frequently prescribed therapeutic classes of medicines are analgesics, antihyperlipidemic agents and antidepressants. Generally, medicines may be classified based on the consumption pattern into two categories such as, short-term consumption and long-term consumption. Medicines prescribed in acute infection cases like antibiotics can be classified as short-term consumption, since the duration of consumption is less, typically in days. However, medicines prescribed to treat and manage chronic cases like tuberculosis or to treat lifestyle-induced diseases such as diabetes, hypertension and the like are usually given over months or even years, based on the underlying condition, and hence can be categorized as long-term consumption.

Traditionally, clinical treatment of chronic diseases or lifestyle-induced diseases involves first checking the vital parameters of the patient. For example, suppose a patient with hypertension is visiting a hospital as a follow-up check-up after a certain period (say 3 months), then as a first step the vital parameters such as blood pressure (BP) is measured along with blood test for cholesterol levels. Based on the BP measurement and blood test reports, the current hypertension dose is evaluated and if needed the dosage is adjusted (higher/lower). This traditional approach considers only discrete parameters values (in this case, BP and cholesterol values measured at hospital) and does not consider variations or daily recordings of the parameters. Moreover, the traditional approach is too subjective with respect to each clinician and the individual practices associated with each clinician. The traditional approach provides no personalized recommendation for each patient. Also, in the traditional approach, the clinician suggests lifestyle-related modifications such as, change in diet, change in activity levels and the like, but the results of the suggested changes are not monitored and considered for better management of diseases.

The information disclosed in this background of the disclosure section is only for enhancement of understanding of the general background of the invention and should not be taken as an acknowledgement or any form of suggestion that this information forms the prior art already known to a person skilled in the art.

SUMMARY OF THE INVENTION

Embodiments of the invention aims to at least partly fulfil the aforementioned needs. To this end, embodiments of the invention provides devices, systems and methods as defined in the independent claims. The dependent claims provide advantageous embodiments.

In an embodiment, the present disclosure may relate to a method for providing personalized recommendation for health management. The method comprises receiving information on medical history, one or more available lifestyle parameters of a plurality of lifestyle parameters and one or more available vital parameters of a plurality of vital parameters, associated with a subject, from one or more devices, estimating at least one of, one or more non-available lifestyle parameters of the plurality of lifestyle parameters and one or more non-available vital parameters of the plurality of vital parameters, for the subject, based on at least one of, the one or more available vital parameters and the one or more available lifestyle parameters, using a trained Neural Network (NN) Classifier. The method comprises predicting at least one of, medicine dosage level, one or more changes to be made in lifestyle parameters, indication to visit a clinician and details on interaction between one or more medications, based on the received information and the estimation, using the trained NN classifier and providing recommendations personalized to the subject, to at least one of the subject and the clinician associated with the subject based on the prediction.

In an embodiment, the present disclosure may relate to a personalized recommendation system for providing personalized recommendation for health management. The personalized recommendation system may comprise a processor and a memory communicatively coupled to the processor, where the memory stores processor executable instructions, which, on execution, may cause the personalized recommendation system to receive information on medical history, one or more available lifestyle parameters of a plurality of lifestyle parameters and one or more available vital parameters of a plurality of vital parameters, associated with a subject, from one or more devices, estimates at least one of, one or more non-available lifestyle parameters of the plurality of lifestyle parameters and one or more non-available vital parameters of the plurality of vital parameters, for the subject, based on at least one of, the one or more available vital parameters and the one or more available lifestyle parameters, using a trained Neural Network (NN) Classifier. The personalized recommendation system predicts at least one of, medicine dosage level, one or more changes to be made in lifestyle parameters, indication to visit a clinician and details on interaction between one or more medications, based on the received information and the estimation, using the trained NN classifier and provides recommendations personalized to the subject, to at least one of the subject and the clinician associated with the subject based on the prediction.

The foregoing summary is illustrative only and is not intended to be in any way limiting. In addition to the illustrative aspects, embodiments, and features described above, further aspects, embodiments, and features will become apparent by reference to the drawings and the following detailed description.

These and other aspects of the invention will be apparent from and elucidated with reference to the embodiment(s) described hereinafter.

BRIEF DESCRIPTION OF THE DRAWINGS

Examples in accordance with aspects of the invention will now be described in detail with reference to the accompanying schematic drawings, in which:

Fig.1 illustrates an exemplary environment for providing personalized recommendation for health management in accordance with some embodiments;
Fig.2 shows a detailed block diagram of a personalized recommendation system in accordance with some embodiments;
Fig.3A shows an exemplary representation for providing personalized recommendation to a subject and clinician in accordance with some embodiments;
Fig.3B show an exemplary representation of interaction between two medicines in accordance with some embodiments;
Fig.4 illustrates a flowchart showing a method for providing personalized recommendation for health management in accordance with some embodiments; and
Fig.5 illustrates a block diagram of an exemplary computer system for implementing some embodiments.

It should be appreciated by those skilled in the art that any block diagrams herein represent conceptual views of illustrative systems embodying the principles of the present subject matter. Similarly, it will be appreciated that any flow charts, flow diagrams, state transition diagrams, pseudo code, and the like represent various processes which may be substantially represented in computer readable medium and executed by a computer or processor, whether or not such computer or processor is explicitly shown.

DETAILED DESCRIPTION OF THE EMBODIMENTS

Certain embodiment of the invention relate to a method and a system for providing personalized recommendation for health management. An embodiment discloses a multi-step approach for providing personalized recommendation for health management. The personalized recommendation system provides recommendation to a subject requesting for personalized recommendation based on at least one of lifestyle and vital parameters of the subject. The personalized recommendation system may estimate at least one of non-available lifestyle parameters and non-available vital parameters associated with the subject based on available lifestyle parameters of a plurality of lifestyle parameters and available vital parameters of a plurality of vital parameters. In an embodiment, the personalized recommendation system may use a neural network (NN) classifier for estimation. Based on the estimation and information on medical history, the available lifestyle parameters and vital parameters received from the subject, at least one of medicine dosage level, one or more changes to be made in lifestyle parameters, indication to visit a clinician and details on interaction between one or more medications may be predicted using the trained NN classifier. Thereby, at least one of the subject and the clinician associated with the subject may be provided with the recommendation, personalized for the subject, based on the prediction. Embodiment of the invention provide personalized recommendation on health to a subject based on lifestyle and vital parameters associated with that subject.

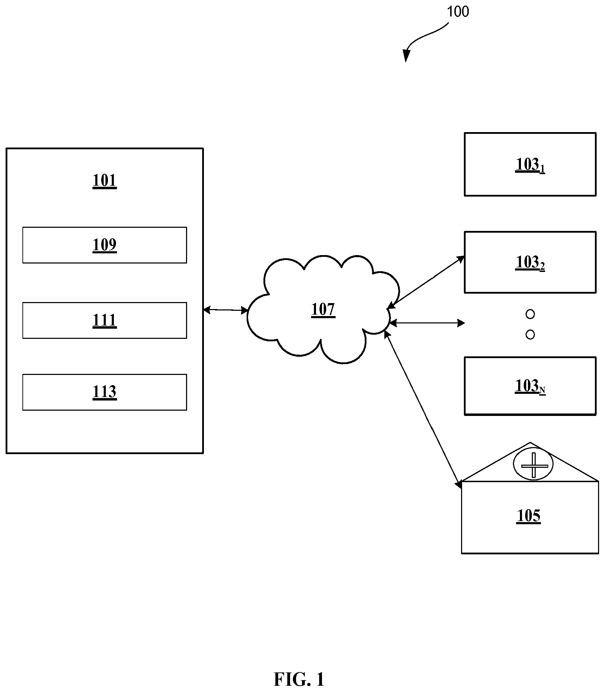

Fig.1 shows an environment 100 comprising a personalized recommendation system 101 connected through a communication network 107 to a subject device $103_1$, a subject device $103_2$, and a subject device $103_N$ (collectively referred as plurality of subject devices 103) associated with a subject and a healthcare system 105.

In an embodiment, the plurality of subject devices 103 may include monitoring devices, screening devices, diagnostic devices and portable and non-portable devices. In an embodiment, the monitoring devices represent home-based devices used for recording vital parameters. In an embodiment, the monitoring devices may include, but are not limited to, BP measuring device, blood glucose measuring device, temperature measuring devices and any other monitoring, screening and diagnostic devices. A person skilled in the art would understand that, any other monitoring devices, not mentioned explicitly, may also be used.

In an embodiment, the portable devices may include, but are not limited to, devices used for measuring lifestyle parameters and vital parameters. In an embodiment, the portable devices may include, but not limited to, mobile devices such as, mobile phones, wireless physical activity tracker and the like. A person skilled in the art would understand that, any other portable devices and non-portables devices, not mentioned explicitly, may also be used for determining lifestyle parameters and vital parameters. The healthcare system 105 may include, but is not limited to, organizations, and trained personnel engaged in providing health care services. In an embodiment, the trained personnel may include clinician. Further, the communication network 107 may include, but is not limited to, a direct interconnection, an e-commerce network, a Peer to Peer (P2P) network, Local Area Network (LAN), Wide Area Network (WAN), wireless network (e.g., using Wireless Application Protocol), Internet, Wi-Fi and the like. The personalized recommendation system 101 provides personalized recommendation to at least one of the subject and to the healthcare system 105 and to the clinician. In an embodiment, the personalized recommendation system 101 may include, but is not limited to, a laptop, a desktop computer, a Personal Digital Assistant (PDA), a notebook, a smartphone, a tablet, a server and any other computing devices. A person skilled in the art would understand that, any other devices, not mentioned explicitly, may also be used as the personalized recommendation system 101.

The personalized recommendation system 101 may receive information on medical history, one or more available lifestyle parameters of a plurality of lifestyle parameters and one or more available vital parameters of a plurality of vital parameters from the subject, whenever a personalized recommendation is required. In an embodiment, the medical history comprises a record of past circumstances relevant to current state of health of the subject, information on past diseases, family medical history, injuries, treatments, medical facts, prescribed medications, and dosage associated with the subject. A person skilled in the art would understand that any other medical details, not mentioned explicitly, may also be present in the present disclosure. In an embodiment, the plurality of lifestyle parameters may include, but are not limited to, social, physical, psychological, and environmental activities associated with the subject. A person skilled in the art would understand that any other lifestyle parameter, not mentioned explicitly, may also be present in the present disclosure. In an embodiment, the plurality of vital parameters comprises status of vital functions associated with body of the subject. For instance, the vital functions may comprise body temperature, pulse rate, blood pressure, respiration rate and the like. A person skilled in the art would understand that any other vital parameters, not mentioned explicitly, may also be present in the present disclosure. In an embodiment, the one or more available lifestyle parameters and the one or more available vital parameters may be measured for a predefined duration. The personalized recommendation system 101 may estimate at least one of one or more non-available lifestyle parameters of the plurality of lifestyle parameters and one or more non-available vital parameters of the plurality of vital parameters for the subject based on at least one of the one or more available lifestyle parameters and the one or more available vital parameters. The estimation may be performed using a Neural Network (NN) classifier. Subsequently, the personalized recommendation system 101 may predict at least one of medicine dosage level, one or more changes to be made in lifestyle parameters, indication to visit a clinician and details on interaction between one or more medications based on the received information and the estimation using the trained NN classifier. Thereafter, the personalized recommendation system 101 may provide recommendation to at least one of the subject and the healthcare system 105 based on the prediction. In an embodiment, the subject is provided with personalized recommendation on at least one of the one or more changes to be made in lifestyle parameters and indication to visit the clinician. In an embodiment, the clinician associated with the subject is provided with recommendation on at least one of the medicine dosage level and the details on interaction between the one or more medicines. In an embodiment, the personalized recommendation system 101 may optimize the personalized recommendation by learning based on previous personalized recommendation along with one or more suggestions provided by the clinician on the recommendation.

The I/O interface 109 may be configured to receive the information on medical history, the one or more available lifestyle parameters and the one or more available vital parameters from the plurality of subject devices 103. The I/O interface 109 may receive suggestions on the personalized recommendation from the clinician associated with the subject. Further, the I/O interface 109 may provide the personalized recommendation to at least one of the subject and the clinician associated with the subject. The information received from the I/O interface 109 may be stored in the memory 111. The memory 111 may be communicatively coupled to the processor 113 of the personalized recommendation system 101. The memory 111 may also store processor instructions which may cause the processor 113 to execute the instructions for providing personalized recommendation on health management.

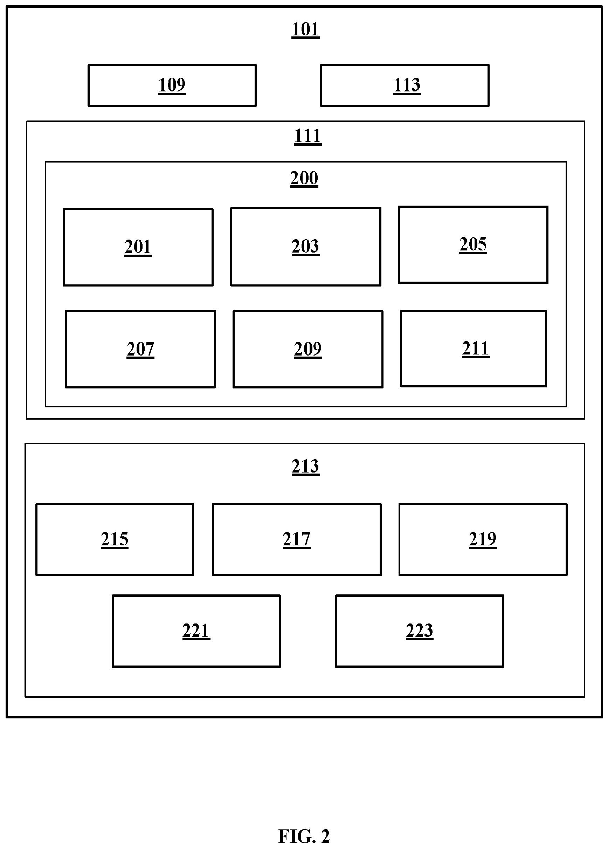

Fig.2 shows a detailed block diagram of a personalized recommendation system in accordance with some embodiments of the present disclosure. Data 200 and one or more modules 213 of the personalized recommendation system 101 are shown. In an embodiment, the data 200 may include medical history data 201, lifestyle data 203, vital data 205, prediction data 207, recommendation data 209 and other data 211. The medical history data 201 may comprise details on past medical details of the subject. The details may comprise record of past circumstances relevant to current state of health of the subject, information on past diseases, injuries, treatments, medical facts, prescribed medications, and dosages for one or more diseases. For example, details of the medical history for a subject is shown in Table 1 below.

Table 1

| MEDICAL HISTORY | |
|---|---|
| Parameters | Description |
| Infectious Diseases | Usual childhood illnesses. No history of rheumatic fever |
| Immunizations | Flu vaccine yearly. Pneumovax 1996 Allergic to Penicillin-developed a diffuse rash after an injection 20 years ago |
| Transfusions | 4 units received in 1980 for GastroIntestinal (GI) hemorrhage, transfusion complicated by Hepatitis B infection. |
| Hospitalizations, Operations, Injuries | 1) Normal childbirth 48 years ago<br>2) 1980 Gastrointestinal haemorrhage.<br>3) 9/1995 chest pain-see history of present illness |
| Vital Parameters | BP: 130/80 on 01/Jan/1980, 11:43 am<br>Pulse Rate: 80 01/Jan/1980, 11:43am |

The lifestyle data 203 may comprise details of the plurality of lifestyle parameters associated with the subject. In an embodiment, the lifestyle parameters may comprise social, physical, psychological, and environmental activities associated with the subject. For instance, the plurality of lifestyle parameters may comprise, diet plan of the subject, physical activities details, sleep quality, stress level, nature of job, environmental parameters such as temperature and the like.

The vital data 205 may comprise details of the plurality of vital parameters associated with the subject. In an embodiment, the plurality of vital parameters comprises status of vital functions associated with body of the subject. For example, the vital functions may comprise, body temperature, blood pressure, cholesterol level, pulse rate, respiration rate and the like. A person skilled in the art would understand that any other vital functions, not mentioned explicitly, may also be used in the present disclosure.

The prediction data 207 may comprise details on at least one of the medicine dosage level, one or more changes to be made in lifestyle parameters, indication to visit the clinician and details on interaction between the one or more medications. For example, the prediction on medicine dosage level may indicate one of lowering dosage level, constant dosage level and higher dosage level of a particular medicine being taken by the subject.

The recommendation data 209 may comprise details on the recommendations personalized to the subject. The recommendations personalized to the subject may be provided to at least the subject and the clinician associated with the subject. For instance, the recommendations such as, information on the one or more changes to be made in lifestyle parameters and time duration at which the subject may be alerted to visit the clinician may be provided to the subject. Further, the personalized recommendation for the subject which may be provided to the clinician may comprise amount of the medicine dosage level and the details on interaction between the one or more medicines, if applicable, may be provided to assist the clinician.

The other data 211 may store data, including temporary data and temporary files, generated by modules 213 for performing the various functions of the personalized recommendation system 101.

In an embodiment, the data 200 in the memory 111 are processed by the one or more modules 213 of the personalized recommendation system 101. As used herein, the term module refers to an application specific integrated circuit (ASIC), an electronic circuit, a field-programmable gate arrays (FPGA), Programmable System-on-Chip (PSoC), a combinational logic circuit, and/or other suitable components that provide the described functionality. The said modules 213 when configured with the functionality defined in the present disclosure will result in a novel hardware.

In one implementation, the one or more modules 213 may include, but are not limited to a receiving module 215, an estimation module 217, a recommendation prediction module 219 and a recommendation providing module 221. The one or more modules 213 may also include other modules 223 to perform various miscellaneous functionalities of the personalized recommendation system 101. In an embodiment, the other modules 223 may include a recommendation optimizing module which may optimize the personalized recommendation by learning based on previous personalized recommendation along with one or more suggestions provided by the clinician on the recommendation.

The receiving module 215 may receive the information on medical history, the one or more available lifestyle parameters and the one or more available vital parameters of the subject from the plurality of subject devices 103. In an embodiment, the subject may determine the one or more available lifestyle parameters and the one or more available vital parameters regularly for the predefined period. In an embodiment, the one or more available lifestyle parameters and the one or more available vital parameters may be determined every day. In an embodiment, the predefined period may comprise one month.

The estimation module 217 may estimate at least one of the one or more non-available lifestyle parameters of the plurality of lifestyle parameters and the one or more non-available vital parameters of the plurality of vital parameters based on at least one of the one or more available lifestyle parameters and the one or more available vital parameters. The estimation module 217 may estimate by using the NN classifier. In an embodiment, the NN classifier may comprise an ensemble of Recurrent Neural Networks (RNN), Convolutional Neural Network (CNN) and Long Short-Term Memory (LSTM) and combination of any of these techniques. In an embodiment, the NN classifier may be composed of multi-stage approach. For instance, $x_1$, $x_2$, $x_3$, $x_4$ and $x_5$ are lifestyle parameters and $y_1$, $y_2$, $y_3$, $y_4$ and $y_5$ are the vital parameters. Consider, $x_1$, $x_2$, $x_4$ and $x_5$ and $y_1$, $y_2$, $y_3$ $y_4$ and $y_5$ as the available lifestyle parameters and the available vital parameters respectively. In such case, the estimation module 217 may estimate value of $x_3$ based on $x_1$, $x_2$, $x_4$ and $x_5$ and $y_1$, $y_2$, $y_3$, $y_4$ and ys. Similarly, one or more unavailable vital parameters may be estimated based on the available vital parameters and the available lifestyle parameters. Thus, the estimation module 217 defines a relationship between the plurality of lifestyle parameters and the plurality of clinical parameters as shown in equation 1.

$$y = \bigcup_i x_i \tag{1}$$

Where, "$x_i$" is $i^{th}$ lifestyle parameter that contributes to changes in clinical parameter.

'y' is vital parameter.

The recommendation prediction module 219 may predict at least one of the medicine dosage level, one or more changes to be made in lifestyle parameters, indication to visit a clinician and details on interaction between one or more medications, based on the received information and the estimation, using the trained NN classifier. In an embodiment, the recommendation prediction module 219 may predict the medicine dosage level based on the received information on the one or more available lifestyle parameters, the one or more available vital parameters, the medical history and the estimated at least one of the one or more non-available lifestyle parameters and the one or more non-available vital parameters. Thus, the recommendation prediction module 219 defines a correlation between medicine dosage, associated vital parameters and lifestyle parameters as shown in equation 2.

$$d_i = f(y_i, x_1, x_2, \ldots \ldots x_n) \tag{2}$$

Where $d_i$ = medicine dosage
$y_i$ = plurality of vital parameters
$x_i$ = plurality of lifestyle parameters.

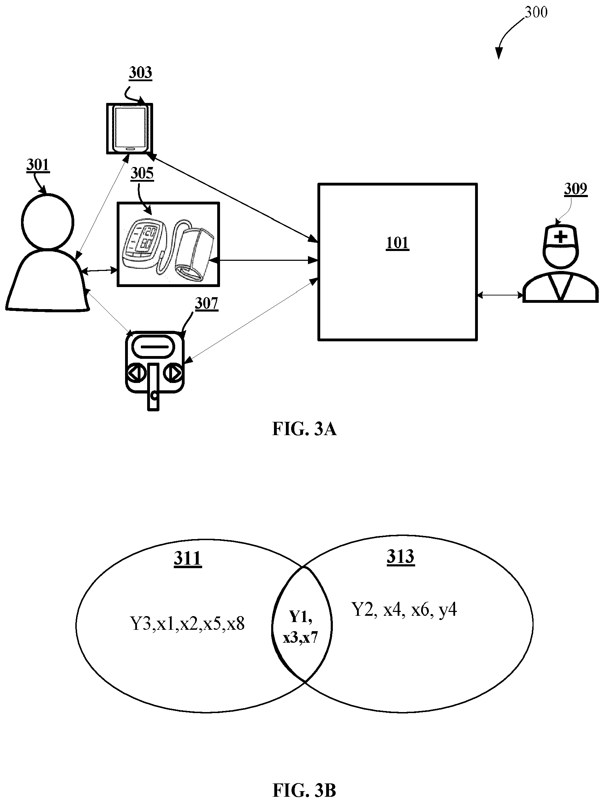

Further, the recommendation prediction module 219 may determine the interaction between the one or more medications, in case the subject consumes various medicines for different diseases. Fig.3B show an exemplary representation of interaction between two medicines in accordance with some embodiments of the present disclosure. Typically, in co-morbid subjects or critically ill subjects, multiple medicines may be prescribed to handle different illness or to manage same disease in multiple ways. The multiple medicines provided to the subject may interact with each other positively or negatively. For instance, consider a subject P, suffering from High Blood Pressure (HBP) and mild cholesterol levels ($C_1$) at initial time ($T_1$). The subject P is monitoring his weight ($X_1$) and exercise parameter such as, calories burned ($X_2$) regularly. The subject P visits clinician at $T_1$ and the clinician prescribes the subject P with a HBP lowering medicine and a composition of Lisinopril medicine (L). The subject P continues to monitor lifestyle parameters $X_1$ and $X_2$ and in addition, the subject P monitors vital parameter such as, blood pressure ($Y_1$). The subject P provides values of the lifestyle parameters such as, X1, X2 and of vital parameter Y1 to the personalized recommendation system 101. The Lisinopril medicine (L) continues to show its effect and maintains blood pressure at a given level. Based on the values of the lifestyle parameters and the vital parameter, the recommendation prediction module 219 may determine that the blood pressure of the subject P is under control, but the cholesterol levels (C1) have increased from mild to severe, denoted as (C2). In such case, the recommendation prediction module 219 may determine a medicine statin (S) for controlling the severe cholesterol level (C2) along with dosage level of the statin (S) for providing to the subject P. At the same time, the recommendation prediction module 219 may determine interference of the medicine (S) with the medicine (L) for providing the interference to the clinician associated with the subject. In an embodiment, the clinician may provide suggestions on the recommendations personalized for the subject.

As shown in fig.3B, interaction between two medicines namely, medicine M1 denoted as 311 and medicine M2 denoted as 313 is shown. The medicine M1 311 is defined as shown in equation 3.

$$M_1 = f(x_1, x_2, x_3, x_5, x_7, x_8, y_1, y_3) \qquad (3)$$

Similarly, the medicine M2 313 is defined as shown in equation 4.

$$M_2 = f(x_3, x_4, x_6, x_7, y_1, y_2, y_4) \qquad (4)$$

As shown in the fig.3B and in the equations 3 and 4, the vital parameter "$y_1$" as well as lifestyle parameters "$x_3$" and "$x_7$" overlap in the medicine M1 311 and medicine M2 313. Therefore, the recommendation prediction module 219 may determine monitoring of these parameters or reverse prediction of these parameters using given medicines to predict the interaction between the two medicines.

The recommendation providing module 221 may provide the recommendation personalized for the subject, to the subject and the clinician associated with the subject, based on the prediction by the recommendation prediction module 219. The recommendation providing module 221 may provide the subject with personalized recommendation on at least one of the one or more changes to be made in lifestyle parameters and indication to visit the clinician. For instance, if the subject is suffering from high blood pressure, the one or more changes to be made in lifestyle may be recommend as less salt intake in food, regular exercise etc. Similarly, based on the prediction, the recommendation providing module 221 may indicate a suggested time to visit the clinician to the subject. Further, the recommendation providing module 221 may provide recommendation to the clinician associated with the subject on at least one of the medicine dosage level and the details on interaction between the one or more medicines to assist the clinician.

Fig.3A shows an exemplary representation for providing personalized recommendation to a subject and clinician in accordance with some embodiments of the present disclosure.

Fig.3B shows an exemplary representation 300 of providing personalized recommendation on health management. The exemplary representation 300 comprises a subject 301 connected to the personalized recommendation system 101 through a mobile phone 303. A person skilled in the art would understand that the subject 301 may be connected to the personalized recommendation system 101 through any other devices, not mentioned explicitly in the present disclosure. The personalized recommendation system 101 is also connected to a clinician 309 associated with the subject 301. At an initial time T1, suppose the subject 301 is suffering from High Blood Pressure (HBP) and is under medication. In such case, the subject 301 may request the personalized recommendation system 101 for personalized recommendation on health. In view of suffering from HBP, the subject 301 may regularly determine the lifestyle parameters such as, salt intake, weight and exercise parameters such as, calories burned for the predefined time using the mobile device 303. For instance, the predefined time may be 1 month. In addition, the subject 301 may monitor vital parameters such as, HBP using a HBP measuring device 305 and cholesterol level using a cholesterol measuring device 307. Consider, that the subject 301 is on travel. The subject 301 is monitoring the lifestyle parameters such as diet plan (Y1) during the travel. Suppose, the subject 301 diet is changed, and the salt intake is increased during travel period. The subject 301 provides the medical history, the one or more available lifestyle parameters, in this case, the diet plan and the one or more available vital parameters to the personalized recommendation system 101. The personalized recommendation system 101 may estimate at least the one or more non-available lifestyle parameters and the one or more non-available vital parameters for the subject 301. In this case, the personalized recommendation system 101 may estimate the vital parameter such as, blood pressure of the subject 301 based on the diet plan and other available vital parameters. Due to change in salt intake, the personalized recommendation system 101 may estimate values of the blood pressure. Further, the personalized recommendation system 101 may predict at least one of the medicine dosage level, the one or more changes to be made in lifestyle parameters, indication to visit a clinician and details on interaction between one or more medications, based on the received information and the estimation. In the present case, the personalized recommendation system 101 may predict change in dosage level for high blood pressure medicine, reduce of salt intake and early warning to visit clinician. Therefore, the personalized recommendation system 101 may provide the personalized recommendation to the subject 301 on the prediction. In the present case, the personalized recommendation to the subject 301 comprise reduce in salt intake and an early visit to the clinician 309. Subsequently, the personalized recommendation system 101 may provide recommendation on the change in the dosage level of high blood pressure medicines of the subject 301 to the clinician 309 associated with the subject 301.

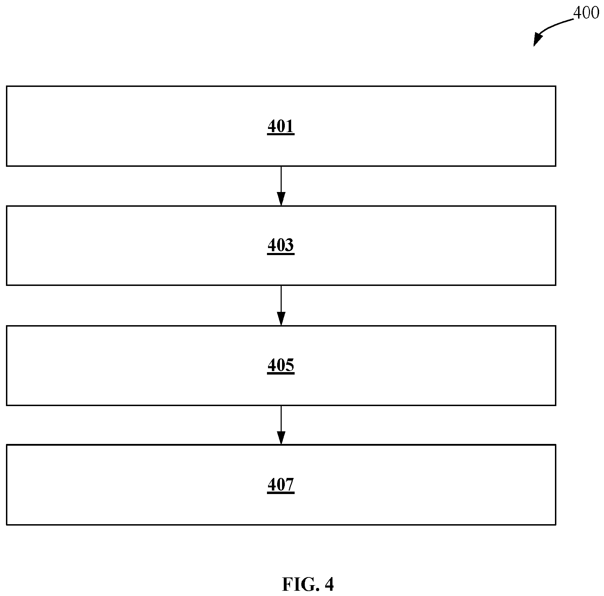

Fig.4 illustrates a flowchart showing a method for providing personalized recommendation for health management in accordance with some embodiments of present disclosure.

As illustrated in Fig.4, the method 400 includes one or more blocks for providing personalized recommendation for health management. The method 400 may be described in the general context of computer executable instructions. Generally, computer executable instructions can include routines, programs, objects, components, data structures, procedures, modules, and functions, which perform particular functions or implement particular abstract data types.

The order in which the method 400 is described is not intended to be construed as a limitation, and any number of the described method blocks can be combined in any order to implement the method. Additionally, individual blocks may be deleted from the methods without departing from the spirit and scope of the subject matter described herein. Furthermore, the method can be implemented in any suitable hardware, software, firmware, or combination thereof.

At block 401, the information on medical history, one or more available lifestyle parameters of the plurality of lifestyle parameters and the one or more available vital parameters of the plurality of vital parameters, associated with the subject are received by the receiving module 215 from the plurality of subject devices 103 associated with the subject.

At block 403, at least one of the one or more non-available lifestyle parameters of the plurality of lifestyle parameters and the one or more non-available vital parameters of the plurality of vital parameters are estimated for the subject, by the estimation module 217 based on at least one of the one or more available vital parameters and the one or more available lifestyle parameters. The estimation module 217 may use the trained Neural Network (NN) Classifier for estimation.

At block 405, at least one of the medicine dosage level, the one or more changes to be made in lifestyle parameters, the indication to visit the clinician and details on interaction between the one or more medications are predicted by the recommendation predication module 217 based on the received information and the estimation using the trained NN classifier.

At block 407, the recommendation is provided, personalized to the subject, by the recommendation providing module 221 to at least one of the subject and the clinician associated with the subject based on the prediction.

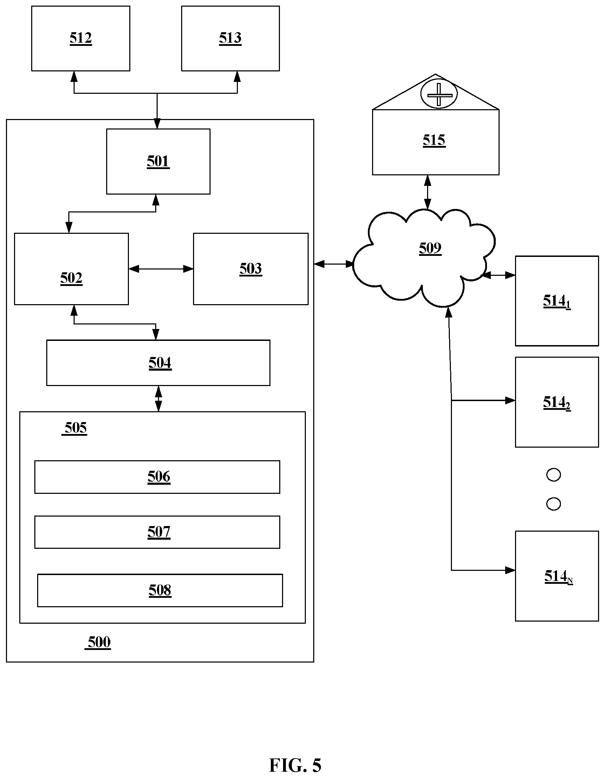

Fig.5 illustrates a block diagram of an exemplary computer system 500 for implementing embodiments consistent with the present disclosure. In an embodiment, the computer system 500 may be used to implement the personalized recommendation system 101. The computer system 500 may include a central processing unit ("CPU" or "processor") 502. The processor 502 may include at least one data processor for providing personalized recommendation for health management. The processor 502 may include specialized processing units such as, integrated system (bus) controllers, memory management control units, floating point units, graphics processing units, digital signal processing units, etc.

The processor 502 may be disposed in communication with one or more input/output (I/O) devices (not shown) via I/O interface 501. The I/O interface 501 may employ communication protocols/methods such as, without limitation, audio, analog, digital, monoaural, RCA, stereo, IEEE-1394, serial bus, universal serial bus (USB), infrared, PS/2, BNC, coaxial, component, composite, digital visual interface (DVI), high-definition multimedia interface (HDMI), RF antennas, S-Video, VGA, IEEE 802.n /b/g/n/x, Bluetooth, cellular (e.g., code-division multiple access (CDMA), high-speed packet access (HSPA+), global system for mobile communications (GSM), long-term evolution (LTE), WiMax, or the like), etc.

Using the I/O interface 501, the computer system 500 may communicate with one or more I/O devices. For example, the input device may be an antenna, keyboard, mouse, joystick, (infrared) remote control, camera, card reader, fax machine, dongle, biometric reader, microphone, touch screen, touchpad, trackball, stylus, scanner, storage device, transceiver, video device/source, etc. The output device may be a printer, fax machine, video display (e.g., cathode ray tube (CRT), liquid crystal display (LCD), light-emitting diode (LED), plasma, Plasma display panel (PDP), Organic light-emitting diode display (OLED) or the like), audio speaker, etc.

In some embodiments, the computer system 500 consists of the personalized recommendation system 101. The processor 502 may be disposed in communication with the communication network 509 via a network interface 503. The network interface 503 may communicate with the communication network 509. The network interface 503 may employ connection protocols including, without limitation, direct connect, Ethernet (e.g., twisted pair 10/100/1000 Base T), transmission control protocol/internet protocol (TCP/IP), token ring, IEEE 802.11a/b/g/n/x, etc. The communication network 509 may include, without limitation, a direct interconnection, local area network (LAN), wide area network (WAN), wireless network (e.g., using Wireless Application Protocol), the Internet, etc. Using the network interface 503 and the communication network 509, the computer system 500 may communicate with a subject device $514_1$, a subject device $514_2$ and a subject device $514_N$ (plurality of subject device 514) and a healthcare system 515. The network interface 503 may employ connection protocols include, but not limited to, direct connect, Ethernet (e.g., twisted pair 10/100/1000 Base T), transmission control protocol/internet protocol (TCP/IP), token ring, IEEE 802.11a/b/g/n/x, etc.

The communication network 509 includes, but is not limited to, a direct interconnection, an e-commerce network, a peer to peer (P2P) network, local area network (LAN), wide area network (WAN), wireless network (e.g., using Wireless Application Protocol), the Internet, Wi-Fi and such. The first network and the second network may either be a dedicated network or a shared network, which represents an association of the different types of networks that use a variety of protocols, for example Hypertext Transfer Protocol (HTTP), Transmission Control Protocol/Internet Protocol (TCP/IP), Wireless Application Protocol (WAP), etc., to communicate with each other. Further, the first network and the second network may include a variety of network devices, including routers, bridges, servers, computing devices, storage devices, etc.

In some embodiments, the processor 502 may be disposed in communication with a memory 505 (e.g., RAM, ROM, etc. not shown in figure 5) via a storage interface 504. The storage interface 504 may connect to memory 505 including, without limitation, memory drives, removable disc drives, etc., employing connection protocols such as, serial advanced technology attachment (SATA), Integrated Drive Electronics (IDE), IEEE-1394, Universal Serial Bus (USB), fiber channel, Small Computer Systems Interface (SCSI), etc. The memory drives may further include a drum, magnetic disc drive, magneto-optical drive, optical drive, Redundant Array of Independent Discs (RAID), solid-state memory devices, solid-state drives, etc.

The memory 505 may store a collection of program or database components, including, without limitation, user interface 506, an operating system 507 etc. In some embodiments, computer system 500 may store user/application data 506, such as, the data, variables, records, etc., as described in this disclosure. Such databases may be implemented as fault-tolerant, relational, scalable, secure databases such as Oracle or Sybase.

The operating system 507 may facilitate resource management and operation of the computer system 500. Examples of operating systems include, without limitation, APPLE MACINTOSH$^R$ OS X, UNIX$^R$, UNIX-like system distributions (E.G., BERKELEY SOFTWARE DISTRIBUTION™ (BSD), FREEBSD™, NETBSD™, OPENBSD™, etc.), LINUX DISTRIBUTIONS™ (E.G., RED HAT™, UBUNTU™, KUBUNTU™, etc.), IBM™ OS/2, MICROSOFT™ WINDOWS™ (XP™, VISTA™/7/8, 10 etc.), APPLE$^R$ IOS™, GOOGLE$^R$ ANDROID™, BLACKBERRY$^R$ OS, or the like.

In some embodiments, the computer system 500 may implement a web browser 508 stored program component. The web browser 508 may be a hypertext viewing application, for example MICROSOFT® INTERNET EXPLORER™, GOOGLE® CHROME™, MOZILLA® FIREFOX™, APPLE® SAFARI™, etc. Secure web browsing may be provided using Secure Hypertext Transport Protocol (HTTPS), Secure Sockets Layer (SSL), Transport Layer Security (TLS), etc. Web browsers 508 may utilize facilities such as AJAX™, DHTML™, ADOBE® FLASH™, JAVASCRIPT™, JAVA™, Application Programming Interfaces (APIs), etc. In some embodiments, the computer system 500 may implement a mail server stored program component. The mail server may be an Internet mail server such as Microsoft Exchange, or the like. The mail server may utilize facilities such as ASP™, ACTIVEX™, ANSI™ C++/C#, MICROSOFT®, .NET™, CGI SCRIPTS™, JAVA™, JAVASCRIPT™, PERL™, PHP™, PYTHON™, WEBOBJECTS™, etc. The mail server may utilize communication protocols such as Internet Message Access Protocol (IMAP), Messaging Application Programming Interface (MAPI), MICROSOFT® exchange, Post Office Protocol (POP), Simple Mail Transfer Protocol (SMTP), or the like. In some embodiments, the computer system 500 may implement a mail client stored program component. The mail client may be a mail viewing application, such as APPLE® MAIL™, MICROSOFT® ENTOURAGE™, MICROSOFT® OUTLOOK™, MOZILLA® THUNDERBIRD™, etc.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include Random Access Memory (RAM), Read-Only Memory (ROM), volatile memory, non-volatile memory, hard drives, CD ROMs, DVDs, flash drives, disks, and any other known physical storage media.

An embodiment of the present disclosure helps in prescribing optimal dosage thereby reducing toxic side effects of medicines.

An embodiment of the present disclosure helps in understanding interaction between medicines for co-morbid patients.

An embodiment of the present disclosure helps care giver and patients to objectively monitor dependent lifestyle parameters for a given clinical disease.

An embodiment of the present disclosure acts as an early warning system when the prediction goes beyond the threshold for specific clinical conditions.

The described operations may be implemented as a method, system or article of manufacture using standard programming and/or engineering techniques to produce software, firmware, hardware, or any combination thereof. The described operations may be implemented as code maintained in a "non-transitory computer readable medium", where a processor may read and execute the code from the computer readable medium. The processor is at least one of a microprocessor and a processor capable of processing and executing the queries. A non-transitory computer readable medium may include media such as magnetic storage medium (e.g., hard disk drives, floppy disks, tape, etc.), optical storage (CD-ROMs, DVDs, optical disks, etc.), volatile and non-volatile memory devices (e.g., EEPROMs, ROMs, PROMs, RAMs, DRAMs, SRAMs, Flash Memory, firmware, programmable logic, etc.), etc. Further, non-transitory computer-readable media include all computer-readable media except for a transitory. The code implementing the described operations may further be implemented in hardware logic (e.g., an integrated circuit chip, Programmable Gate Array (PGA), Application Specific Integrated Circuit (ASIC), etc.).

Still further, the code implementing the described operations may be implemented in "transmission signals", where transmission signals may propagate through space or through a transmission media, such as, an optical fiber, copper wire, etc. The transmission signals in which the code or logic is encoded may further include a wireless signal, satellite transmission, radio waves, infrared signals, Bluetooth, etc. The transmission signals in which the code or logic is encoded is capable of being transmitted by a transmitting station and received by a receiving station, where the code or logic encoded in the transmission signal may be decoded and stored in hardware or a non-transitory computer readable medium at the receiving and transmitting stations or devices. An "article of manufacture" includes non-transitory computer readable medium, hardware logic, and/or transmission signals in which code may be implemented. A device in which the code implementing the described embodiments of operations is encoded may include a computer readable medium or hardware logic. Of course, those skilled in the art will recognize that many modifications may be made to this configuration without departing from the scope of the invention, and that the article of manufacture may include suitable information bearing medium known in the art.

The terms "an embodiment", "embodiment", "embodiments", "the embodiment", "the embodiments", "one or more embodiments", "some embodiments", and "one embodiment" mean "one or more (but not all) embodiments of the invention(s)" unless expressly specified otherwise.

The terms "including", "comprising", "having" and variations thereof mean "including but not limited to", unless expressly specified otherwise.

The enumerated listing of items does not imply that any or all of the items are mutually exclusive, unless expressly specified otherwise.

The terms "a", "an" and "the" mean "one or more", unless expressly specified otherwise.

A description of an embodiment with several components in communication with each other does not imply that all such components are required. On the contrary, a variety of optional components are described to illustrate the wide variety of possible embodiments of the invention.

When a single device or article is described herein, it will be readily apparent that more than one device/article (whether or not they cooperate) may be used in place of a single device/article. Similarly, where more than one device or article is described herein (whether or not they cooperate), it will be readily apparent that a single device/article may be used in place of the more than one device or article or a different number of devices/articles may be used instead of the shown number of devices or programs. The functionality and/or the features of a device may be alternatively embodied by one or more other devices which are not explicitly described as having such functionality/features. Thus, other embodiments of the invention need not include the device itself.

The illustrated operations of Figures 4 show certain events occurring in a certain order. In alternative embodiments, certain operations may be performed in a different order, modified or removed. Moreover, steps may be added to the above described logic and still conform to the described embodiments. Further, operations described herein may occur sequentially or certain operations may be processed in parallel. Yet further, operations may be performed by a single processing unit or by distributed processing units.

The description has been presented for purposes of illustration and description, and is not intended to be exhaustive or limited to the invention in the form disclosed. Many modifications and variations will be apparent to those of ordinary skill in the art. Embodiments have been chosen and described in order to best explain principles of proposed embodiments, practical application(s), and to enable others of ordinary skill in the art to understand various embodiments with various modifications are contemplated.

**Claims**

1. A method for providing personalized recommendation for health management, the method comprising:

   receiving, by a personalized recommendation system, information on medical history, one or more available lifestyle parameters of a plurality of lifestyle parameters and one or more available vital parameters of a plurality of vital parameters, associated with a subject, from one or more devices;
   estimating, by the personalized recommendation system, at least one of, one or more non-available lifestyle parameters of the plurality of lifestyle parameters and one or more non-available vital parameters of the plurality of vital parameters, for the subject, based on at least one of, the one or more available vital parameters and the one or more available lifestyle parameters, using a trained Neural Network (NN) Classifier;
   predicting, by the personalized recommendation system, at least one of, medicine dosage level, one or more changes to be made in lifestyle parameters, indication to visit a clinician and details on interaction between one or more medications, based on the received information and the estimation, using the trained NN classifier; and
   providing, by the personalized recommendation system, recommendations personalized to the subject, to at least one of the subject and the clinician associated with the subject based on the prediction.

2. The method as claimed in claim 1, wherein the medical history comprises a record of past circumstances relevant to current state of health of the subject, information on past diseases, injuries, treatments, medical facts, prescribed medications, and dosage.

3. The method as claimed in claim 1, wherein the plurality of lifestyle parameters comprises social, physical, psychological, and environmental activities associated with the subject.

4. The method as claimed in claim 1, wherein the plurality of vital parameters comprises status of vital functions associated with body of the subject.

5. The method as claimed in claim 1, wherein the subject is provided with personalized recommendation on at least one of the one or more changes to be made in lifestyle parameters and indication to visit the clinician.

6. The method as claimed in claim 1, wherein the clinician associated with the subject is provided with recommendation on at least one of the medicine dosage level and the details on interaction between the one or more medicines to assist the clinician.

7. The method as claimed in claim 1 further comprising optimizing the personalized recommendation by learning based on previous personalized recommendation along with one or more suggestion provided by the clinician on the recommendation.

8. A personalized recommendation system for providing personalized recommendation for health management, comprising:

a processor; and
a memory communicatively coupled to the processor, wherein the memory stores processor instructions, which, on execution, causes the processor to:

receive information on medical history, one or more available lifestyle parameters of a plurality of lifestyle parameters and one or more available vital parameters of a plurality of vital parameters, associated with a subject, from one or more devices;
estimate at least one of, one or more non-available lifestyle parameters of the plurality of lifestyle parameters and one or more non-available vital parameters of the plurality of vital parameters, for the subject, based on at least one of, the one or more available vital parameters and the one or more available lifestyle parameters, using a trained Neural Network (NN) Classifier;
predict at least one of, medicine dosage level, one or more changes to be made in lifestyle parameters, indication to visit a clinician and details on interaction between one or more medications, based on the received information and the estimation, using the trained NN classifier; and
provide recommendations personalized to the subject, to at least one of the subject and the clinician associated with the subject based on the prediction.

9. The personalized recommendation system as claimed in claim 9, wherein the medical history comprises a record of past circumstances relevant to current state of health of the subject, information on past diseases, injuries, treatments, medical facts, prescribed medications and dosage.

10. The personalized recommendation system as claimed in claim 9, wherein the plurality of lifestyle parameters comprises social, physical, psychological and environmental activities associated with the subject.

11. The personalized recommendation system as claimed in claim 9, wherein the plurality of vital parameters comprises status of vital functions associated with body of the subject.

12. The personalized recommendation system as claimed in claim 9, wherein the subject is provided with personalized recommendation on at least one of the one or more changes to be made in lifestyle parameters and indication to visit the clinician.

13. The personalized recommendation system as claimed in claim 9, wherein the clinician associated with the subject is provided with recommendation on at least one of the medicine dosage level and interaction between the one or more medicines to assist the clinician.

14. The personalized recommendation system as claimed in claim 9, wherein the processor optimizes the personalized recommendation by learning based on previous personalized recommendation along with one or more suggestion provided by the clinician on the recommendation.

100

101

109

111

113

$103_1$

107

$103_2$

$103_N$

105

**FIG. 1**

**FIG. 2**

300

303

301

305

101

309

307

**FIG. 3A**

311

313

Y3,x1,x2,x5,x8

**Y1,
x3,x7**

Y2, x4, x6, y4

**FIG. 3B**

400

| 401 |
|:---:|

| 403 |
|:---:|

| 405 |
|:---:|

| 407 |
|:---:|

**FIG. 4**

**FIG. 5**

Europäisches
Patentamt

European
Patent Office

Office européen
des brevets

## EUROPEAN SEARCH REPORT

Application Number

EP 18 17 1471

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 6 658 396 B1 (TANG SHARON S [US] ET AL) 2 December 2003 (2003-12-02) * the whole document * * in particular * * column 4, line 56 - column 11, line 6; claims 1-3,5,6,; figures 1,5, 11b, 13 * ----- | 1-14 | INV. G16H50/20 |
| X | US 2003/140063 A1 (PIZZORNO JOSEPH E [US] ET AL) 24 July 2003 (2003-07-24) * the whole document * * in particular * * paragraph [0007] - paragraph [0015]; figures 4, 5, 10I, 10J * * paragraph [0047] - paragraph [0053] * ----- | 1-14 | |

TECHNICAL FIELDS
SEARCHED     (IPC)

G16H

The present search report has been drawn up for all claims

| Place of search | Date of completion of the search | Examiner |
|---|---|---|
| Munich | 30 October 2018 | Rákossy, Zoltán |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

 ......................................................................

& : member of the same patent family, corresponding
  document

EPO FORM 1503 03.82 (P04C01)

**EP 3 567 601 A1**

## ANNEX TO THE EUROPEAN SEARCH REPORT
## ON EUROPEAN PATENT APPLICATION NO.

EP 18 17 1471

30-10-2018

| Patent document cited in search report | | Publication date | Patent family member(s) | Publication date |
|---|---|---|---|---|
| US 6658396 | B1 | 02-12-2003 | NONE | |
| US 2003140063 | A1 | 24-07-2003 | NONE | |